# FASCICULE DE BREVET EUROPEEN

(11) **EP 3 253 299 B1**
(45) Date de publication et mention de la délivrance du brevet: **08.07.2020**
(21) Numéro de dépôt: 16704690.3
(22) Date de dépôt: 05.02.2016
(51) Int. Cl.: A61B 17/04

(54) **SYSTÈME D'EXPANSION CUTANÉE**
HAUTEXPANSIONSSYSTEM
SKIN EXPANSION SYSTEM

(30) Priorité: 06.02.2015 FR 1550937
(43) Date de publication de la demande: 13.12.2017
(73) Titulaire: Medical Innovation Developpement, 69570 Dardilly (FR)
(72) Inventeur: BEATRIX, Olivier, 69005 Lyon (FR)
(74) Mandataire: Plasseraud IP
(86) Numéro de dépôt international: PCT/FR2016/050257
(87) Numéro de publication internationale: WO 2016/124872

(56) Documents cités:
- WO-A1-99/35974
- WO-A1-2013/134767
- JP-A- 2013 094 409
- US-A1- 2003 092 969
- US-A1- 2004 260 344
- US-A1- 2011 040 325
- US-A1- 2012 016 384

## Description

L'invention se rapporte à un système d'expansion cutanée.

En particulier, l'invention se rapporte à un système d'expansion cutanée de type à fermeture à boucle vasculaire (*vessel loop closure*) utilisant une extension tissulaire externe par lien élastique pour réduire une plaie en exerçant une traction le long des tissus bordant la plaie. L'extension tissulaire externe par lien élastique est connue et décrite depuis plusieurs décennies et notamment dans les publications suivantes :
- Harris I, « Graduai closure of fasciotomy wounds using a vessel loop shoelace », Injury 1993 ; 24(8) : 565-6,
- AsgariMM, Spinelli HM, « The vessel loop shoelace technique for closure of fasciotomy wounds », Ann Plast Surg 1999 ; 43 : 225-9,
- Bashir AH, « Wound closure by skin traction: an application of tissue expansion », Br J Plast Surg 1987 ; 40(6) : 582-7,
- Bulstrode CJ, « A simple method for closing fascitomies », Ann R ColSurg Engl 1985 ; 67(2) : 119-20,
- Almekinders LC, « Gradual closure of fasciotomy », Orthop Rev 1991 ; 20(1) : 82-4,
- Monnier J, Extension tissulaire uni-axiale dans la couverture des pertes de substance des membres », Annal chir plast 52 (2007) : 577-581.

Cette technique utilise les propriétés élastiques de la peau notamment et consiste à réaliser une traction progressive sur deux berges cutanées bordant la plaie lorsque la suture directe est impossible du fait d'une perte de substance, d'une tension excessive entre les deux berges ou lors des aponévrotomies de décharge réalisées en cas de syndrome des loges. Ce dernier se définit comme une souffrance neuromusculaire pouvant aller jusqu'à la nécrose, liée à une augmentation de la pression intratissulaire dans une loge musculaire inextensible. Il est secondaire à un traumatisme ou d'une ischémie. La seule façon de faire baisser la pression dans la loge est de l'ouvrir largement. Ce geste est réalisé en chirurgie vasculaire ou chirurgie orthopédique, il permet de sauver le muscle concerné et sa fonction, mais il provoque une large béance cutanée équivalente à une perte de substance.

L'invention s'applique donc au traitement d'une plaie de grande dimension ayant, par exemple, une surface comprise entre 100 cm² et 500 cm² et résultant, par exemple, d'une intervention d'aponévrotomie ou d'une ablation d'une tumeur cutanée. L'invention s'applique également au traitement d'une plaie de plus petite dimension ayant, par exemple, une surface inférieure à 100 cm², résultant, par exemple, d'une désunion post-opératoire.

Le principe de l'extension tissulaire externe est de réaliser une traction progressive sur les deux berges cutanées afin de les rapprocher et idéalement de les mettre en contact avec une plaie alors complètement fermée. Le système d'expansion cutanée est mis en place en fin d'intervention. La traction progressive est généralement réalisée dans les suites immédiates de l'intervention (deuxième jour post opératoire) et poursuivie pendant une quinzaine de jours. On augmente la traction toutes les quarante-huit heures au lit du patient. Le système d'expansion cutanée est ôté sans anesthésie.

Actuellement, cette technique est réalisée grâce à du matériel non spécifique, détourné de son usage initial et en particulier par :
- un lac de silicone comme lien déformable élastiquement destiné à s'étendre entre les tissus bordant la plaie de telle manière qu'une tension dans le lien produise une traction le long des tissus bordant la plaie pour rapprocher lesdits tissus les uns des autres ; le lac silicone est alors détourné de son domaine d'utilisation qui est le repérage per opératoire des structures vasculaires ou nobles (uretère, cholédoque),
- des agrafes servant habituellement à la fermeture cutanée en fin d'intervention,
- un tendeur adapté pour maintenir une tension dans le lien.

Dans ce système d'expansion cutanée connu, le lien est passé successivement dans les agrafes, ancrées au travers des tissus bordant la plaie. Le lien est ensuite maintenu en tension par le tendeur, par exemple sous la forme d'un taquet sur une ou plusieurs des agrafes.

Toutefois, bien que très répandue et éthique car utilisée en situation de sauvetage où il n'existe aucune autre solution alternative, la technique d'extension cutanée, née du détournement de matériels médicaux, présente souvent des résultats décevants.

En particulier, le système d'expansion cutanée connu est long et complexe à mettre en œuvre du fait de la nécessité d'ancrer préalablement les agrafes le long des tissus bordant la plaie. De plus, dans ce système d'expansion cutané, la traction sur les tissus bordant la plaie est exercée par l'intermédiaire des agrafes et dépend de l'ancrage de chacune des agrafes. Malgré le soin apporté à l'ancrage des agrafes, Elles ne permettent pas d'exercer une traction maîtrisée, en particulier selon une orientation appropriée. Le système d'expansion cutanée connu ne permet donc pas d'assurer une uniformité de la traction le long des tissus bordant la plaie. Il existe alors, au moins localement et notamment aux emplacements où la traction est la plus forte, un risque de détérioration voire de nécrose des tissus. De plus, les agrafes détournées de leur usage habituel ne présentent aucune qualité mécanique particulière et risquent de s'arracher.

D'autres exemples de système d'expansion cutanée selon le préambule de la revendication 1 sont décrits dans les documents US 2012/0016384 et US 2003/0092969. De par les ancrages envisagés, ces systèmes d'expansion cutanée présentent toutefois un risque important de détérioration voire de déchirement des tissus bordant la plaie.

L'invention vise à pallier les problèmes évoqués ci-dessus.

A cet effet, l'invention propose un système d'expansion cutanée tel que défini dans la revendication 1.

Ainsi, le lien suturant directement la plaie peut être mis en place de manière simple et rapide par l'intermédiaire d'un geste intuitif pour un chirurgien. Par ailleurs, la traction exercée par le lien déformable élastiquement directement sur la peau peut être mieux répartie entre les points de suture et plus uniforme le long des tissus bordant la plaie de sorte que les risques de détérioration et de nécrose sont réduits. Cette protéger des tissus bordant la plaie par rapport aux risques de détérioration et de nécrose est améliorée par la combinaison du lien extensible élastiquement avec une ou plusieurs cales permettant une reprise de la traction exercée sur les tissus bordant la plaie par une zone superficielle étendue correspondant à la surface d'appui. En particulier, lorsqu'une tension est appliquée sur le lien en vue de refermer la plaie, la traction sur les tissus peut être appliquée de manière progressive, du fait de l'étirement du lien, et répartie, du fait de l'appui de la cale. Les cales peuvent également permettre d'améliorer le guidage du lien et de faciliter sa mise en place.

Le système d'expansion cutanée selon l'invention présente, en outre, notamment les avantages suivants :
- un amarrage cutané solide permettant de mener à terme la procédure,- une protection cutanée permettant d'exercer la traction sans dégât sur la peau,
- le développement d'un système de verrouillage réversible facilement utilisable sur le lac élastique,- une bonne ergonomie alliant une facilité de mise en place, de manipulation et d'ablation,- un système fiable pour une sécurité maximum du patient.

Il offre une amélioration du service médical rendu conforter la technique d'extension cutanée externe dans ses indications classiques actuelles et notamment :
- en chirurgie vasculaire pour fermer des aponévrotomies de décharge après syndrome des loges post ischémie,
- en chirurgie orthopédique pour fermer des aponévrotomies de décharge après syndrome des loges post traumatiques et/ou ischémiques.
- en chirurgie oncologique (cancérologique) pour fermer des grandes pertes de substance cutanées et sous cutanées.

Le système d'expansion cutanée selon l'invention présente par ailleurs une adaptabilité permettant aussi d'étendre les indications de la technique d'extension cutanée externe à de nouvelles indications, telles que :
- la réduction de la surface à greffer après résection de petite tumeur ou reprise élargie dans la prise en charge des mélanomes,
- les désunions cicatricielles post opératoires.

Le lien peut être réalisé en polymère, et notamment en silicone.

Le tendeur peut comprendre :
- un corps solidarisé à la deuxième extrémité du lien, et
- un organe de blocage déplaçable par rapport au corps entre un état inactif dans lequel ledit organe de blocage libère un passage pour le lien entre une portion de blocage dudit organe de blocage et le corps, et un état actif dans lequel ledit organe de blocage maintient une portion du lien à distance de la deuxième extrémité entre la portion de blocage dudit organe de blocage et le corps.

Une telle cale pourrait être utilisée indépendamment du système d'expansion cutané tel que défini précédemment pour toute autre application mettant en œuvre une suture, avec tout type de lien approprié éventuellement différent du lien défini précédemment.

La surface d'appui peut être dépourvue d'élément d'ancrage adapté pour percer les tissus bordant la plaie et pénétrer dans lesdits tissus.

Les cales ne constituent alors pas un point d'ancrage, le point d'ancrage étant constitué par le point de suture du lien au travers des tissus.

La surface de guidage s'étend selon un axe de guidage et présente une concavité autour de l'axe de guidage.

La cale peut comporter au moins une ouverture de guidage présentant une surface latérale autour d'un axe central et adaptée pour permettre un passage du lien, la surface latérale formant au moins une partie de la surface de guidage.

La cale peut comprendre au moins deux ouvertures de guidage séparées l'une de l'autre par une portion sécable.

La cale peut être réalisée en polymère, et notamment en silicone. Ces dispositions permettent de favoriser l'adaptation anatomique de la cale aux tissus sur lesquels elle repose.

Selon un autre aspect, il est décrit un procédé de réduction d'une plaie bordée par des tissus mettant en œuvre un système d'expansion cutanée tel que défini précédemment, le procédé de réduction d'une plaie comprenant les étapes consistant à :
- suturer la plaie en traversant directement les tissus bordant la plaie en au moins deux points de suture avec le lien de telle manière que le lien s'étende entre les tissus bordant la plaie,
- exercer une tension dans le lien de manière à produire une traction le long des tissus bordant la plaie pour rapprocher lesdits tissus les uns des autres,
- maintenir la tension dans le lien avec le tendeur.

Le procédé de réduction d'une plaie peut prévoir en outre d'ajuster, et notamment d'augmenter, la tension dans le lien.

Le procédé de réduction d'une plaie peut prévoir, au cours de l'étape consistant à suturer la plaie, d'interposer au moins une cale entre le lien et les tissus bordant la plaie, la cale présentant une surface d'appui reposant sur les tissus bordant la plaie, et une surface de guidage opposée à la surface d'appui et recevant une portion du lien.

D'autres objets et avantages de l'invention apparaîtront à la lecture de la description qui suit d'un mode de réalisation particulier de l'invention donné à titre d'exemple non limitatif, la description étant faite en référence aux dessins annexés dans lesquels :
- la figure 1 est une représentation en perspective d'un système d'expansion cutanée selon un mode de réalisation de l'invention, le système d'expansion cutanée comprenant un lien adapté pour suturer la plaie, une aiguille de suture solidarisée à une première extrémité du lien et un tendeur solidarisé à une deuxième extrémité du lien,
- la figure 2 est une représentation en coupe selon l'orientation référencée II-II sur la figure 1 du lien du système d'expansion cutanée de la figure 1,
- la figure 3 est une représentation en coupe selon l'orientation référencée III-III sur la figure 1 du tendeur du système d'expansion cutanée de la figure 1,
- les figures 4 et 5 sont des représentations respectivement en perspective et en élévation d'une variante du tendeur du système d'expansion cutanée de la figure 1,
- la figure 6 est une représentation en perspective d'une cale selon un premier mode de réalisation pouvant être mise en œuvre dans le système d'expansion cutanée de la figure 1, la cale étant destinée à être interposée entre le lien et les tissus bordant la plaie,
- la figure 7 est une représentation en perspective d'une variante de la cale de la figure 6,
- les figures 8 et 9 sont des représentations schématiques illustrant la mise en place de la cale de la figure 7 sur des tissus bordant une plaie,
- la figure 10 est une représentation schématique d'une réduction d'une plaie mettant en œuvre le système d'expansion cutanée de la figure 1 avec plusieurs cales des figures 6 et 7 dans un montage en pont,
- la figure 11 est une représentation schématique d'une réduction d'une plaie mettant en œuvre le système d'expansion cutanée de la figure 1 avec plusieurs cales des figures 6 et 7 dans un montage en lacet,
- la figure 12 est une représentation en perspective d'une cale selon un deuxième mode de réalisation pouvant être mise en œuvre dans le système d'expansion cutanée de la figure 1,
- la figure 13 est une représentation en coupe de la cale de la figure 12.

Sur les figures, les mêmes références désignent des éléments identiques ou analogues.

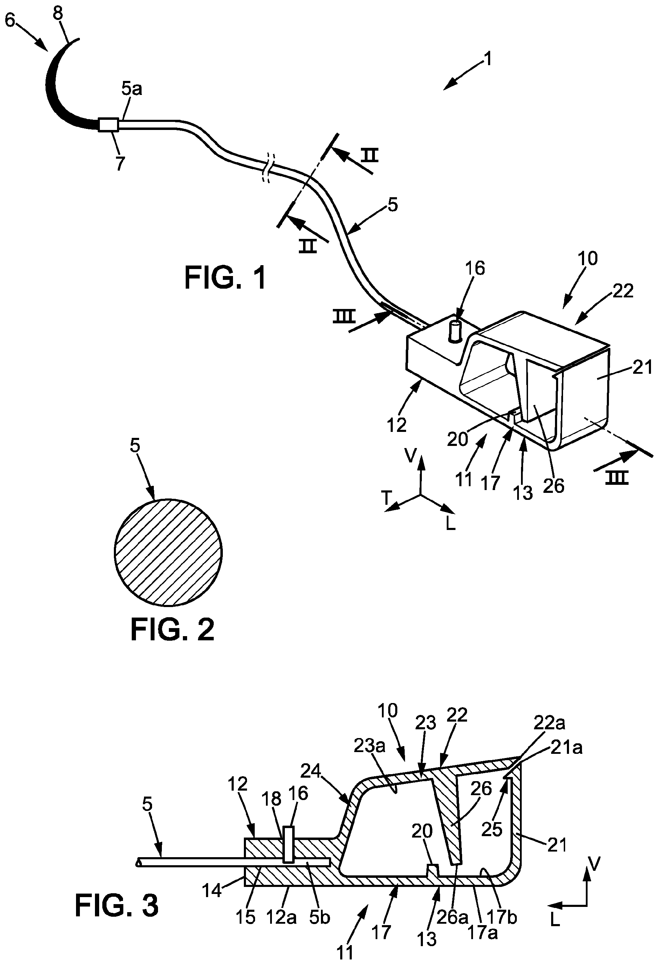

Les figures 1 à 3 représentent un système d'expansion cutanée 1 pour réduire une plaie 2 bordée par des tissus 3. Le système d'expansion cutanée 1 est de type à fermeture à boucle vasculaire (*vessel loop closure*) dans lequel la plaie 2 est réduite en exerçant une traction le long des tissus 3 bordant la plaie 2.

Le système d'expansion cutanée 1 comprend un lien 5 adapté pour suturer la plaie 2 en traversant directement les tissus 3 bordant la plaie en plusieurs points de suture 4. En particulier, le lien 5 est réalisé en un matériau biocompatible et déformable élastiquement et, en particulier, extensible élastiquement, tel que du silicone. Le lien 5 présente, au repos, c'est-à-dire en l'absence de contrainte extérieure, une longueur entre des première 5a et deuxième 5b extrémités opposées et peut être étiré de manière à écarter les première 5a et deuxième 5b extrémités l'une de l'autre. En particulier, la longueur du lien 5 peut être comprise entre 20 cm et 80 cm, de préférence entre 40 cm et 60 cm. Le lien 5 peut présenter une graduation sur toute sa longueur.

Sur la figure 2, le lien 5 est représenté avec une section transversale circulaire de diamètre compris entre 1 et 3 mm. En variante, le lien 5 pourrait présenter toute autre section transversale non circulaire et présentant, par exemple, des première et deuxième dimensions respectivement selon des première D1 et deuxième D2 directions perpendiculaires entre elles telles que la première dimension soit supérieure à la deuxième dimension. En particulier, le lien pourrait avoir une section transversale ovale présentant un grand axe a selon la première direction D1 et un petit axe b selon la deuxième direction D2.

Le lien 5 comprend également une aiguille de suture 6 solidarisée à sa première extrémité 5a. L'aiguille de suture 6 est, par exemple, courbe et peut être sertie sur la première extrémité 5a du lien 5. Une bague 7, solidaire d'une extrémité opposée à une pointe 8 de l'aiguille de suture 6 et adaptée pour recevoir la première extrémité 5a du lien 5, est alors déformée pour retenir le lien 5 à l'intérieur de la bague 7. Pour pouvoir séparer l'aiguille de suture 6 du lien 5 une fois la suture réalisée, l'un des éléments choisi parmi le lien 5 et l'aiguille de suture 6 peut comprendre une portion sécable. En particulier, du fait de la nature du matériau du lien 5, celui-ci peut être coupé à proximité de l'aiguille de suture 6. En variante, l'aiguille de suture 6 peut être solidarisée à la première extrémité 5a du lien 5 par tout moyen approprié et notamment par emmanchement à force, surmoulage ou par l'intermédiaire de tout dispositif de fixation approprié, éventuellement amovible.

La deuxième extrémité 5b du lien 5 est solidarisée à un tendeur 10 adapté pour maintenir une tension dans le lien 5.

Dans le mode de réalisation représenté sur les figures 1 et 3, le tendeur 10 comprend un corps 11 qui s'étend selon une direction longitudinale L entre une partie distale 12 à laquelle le lien 5 est solidarisé, et une partie proximale 13.

La partie distale 12, par exemple de forme parallélépipédique, présente une surface d'extrémité transversale 14 par rapport à la direction longitudinale L et pourvue d'un orifice 15 qui s'étend selon un axe et qui est adapté pour loger la deuxième extrémité 5b du lien 5. Pour maintenir la deuxième extrémité 5b du lien 5 dans l'orifice 15, un organe de serrage 16 est monté dans un trou 18 sur une surface supérieure de la partie distale 12 du corps 11 de manière déplaçable perpendiculairement à l'axe de l'orifice 15. L'organe de serrage 16 peut alors être déplacé entre :
- une position de serrage dans laquelle une partie de l'organe de serrage 16 s'étend dans l'orifice 15 pour pouvoir coincer le lien 5 entre une extrémité de l'organe de serrage 16 et une surface latérale de l'orifice 15, et
- une position escamotée dans laquelle l'organe de serrage 16 ne s'étend pas dans l'orifice 15 de manière à ne pas interférer avec le lien 5.

En variante, la deuxième extrémité 5b du lien 5 pourrait être maintenue dans l'orifice 15 par tout autre moyen approprié.

La partie proximale 13 s'étend selon la direction longitudinale L depuis une surface opposée à la surface d'extrémité 14 de la partie distale 12. Elle est ajoutée selon une direction transversale T, perpendiculaire à la direction longitudinale L. Elle comprend, en particulier, une paroi de base 17 dont une surface inférieure 17a s'étend dans le prolongement d'une surface inférieure 12a de la partie distale 12. La paroi de base 17 comporte, sur une surface supérieure 17b opposée à la surface inférieure 17a, une saillie 20 qui s'étend sur toute sa largeur mesurée selon la direction transversale T. La partie proximale 13 comprend également une paroi transversale 21 qui s'étend perpendiculairement à la direction longitudinale L, selon une direction verticale V, depuis une extrémité de la paroi de base 17 opposée à la partie distale 12.

Le tendeur 10 comprend également un organe de blocage réalisé sous la forme d'un levier de blocage 22 et adapté pour bloquer de manière réversible une portion du lien 5 à distance de la deuxième extrémité 5b.

Dans le mode de réalisation représenté, le levier de blocage 22 est venu de matière avec le corps 11 réalisé en matière plastique. Le levier de blocage 22 est, par ailleurs, conformé pour qu'au repos, en l'absence de contrainte extérieure, une extrémité libre 22a du levier de blocage 22 soit disposée à proximité d'une extrémité libre 21a de la paroi transversale 21 de la partie proximale 13 du corps 11.

En particulier, le levier de blocage 22 comprend une paroi de blocage 23 agencée sensiblement en regard de la paroi de base 17 de la partie proximale 13, et comprenant l'extrémité libre 22a du levier de blocage 22. La paroi de blocage 23 est reliée à la partie distale 12 par une paroi de liaison 24 sensiblement perpendiculaire à la direction longitudinale L. Au repos, le levier de blocage 22 est dans une position ouverte correspondant à un état inactif dans lequel son extrémité libre 22a est située au voisinage de l'extrémité libre 21a de la paroi transversale 21 de la partie proximale 13 du corps 11 en ménageant un passage pour le lien 5.

Le levier de blocage 22 peut être déformé élastiquement pour être déplacé vers une position fermée correspondant à un état actif dans lequel il maintient une portion du lien 5 à distance de la deuxième extrémité 5b entre son extrémité libre 22a formant une portion de blocage et l'extrémité libre 21a de la paroi transversale 21 de la partie proximale 13 du corps 11. L'extrémité libre 21a de la paroi transversale 21 de la partie proximale 13 du corps 11 est alors pourvue d'un ergot 25 pour pouvoir maintenir le levier de blocage 22 à l'état actif, en position fermée.

Le levier de blocage 22 comporte une patte de blocage 26 qui s'étend perpendiculairement depuis une surface inférieure 23a de la paroi de blocage 23 en regard de la surface supérieure 17b de la paroi de base 17 de la partie proximale 13 du corps 11. La patte de blocage 26 est agencée pour que :
- en position ouverte du levier de blocage 22 à l'état inactif, une extrémité libre 26a de la patte de blocage 26 soit à distance de la saillie 20 de la paroi de base 17 et de la surface supérieure 17b de la paroi de base 17 de manière à permettre au lien 5 de passer,
- en position fermée du levier de blocage 22 à l'état actif, l'extrémité libre 26a de la patte de blocage 26 soit à proximité de la saillie 20 de la paroi de base 17 et de la surface supérieure 17b de la paroi de base 17 de manière à bloquer une portion du lien 5, l'extrémité libre 26a de la patte de blocage 26 formant une autre portion de blocage du levier de blocage 22.

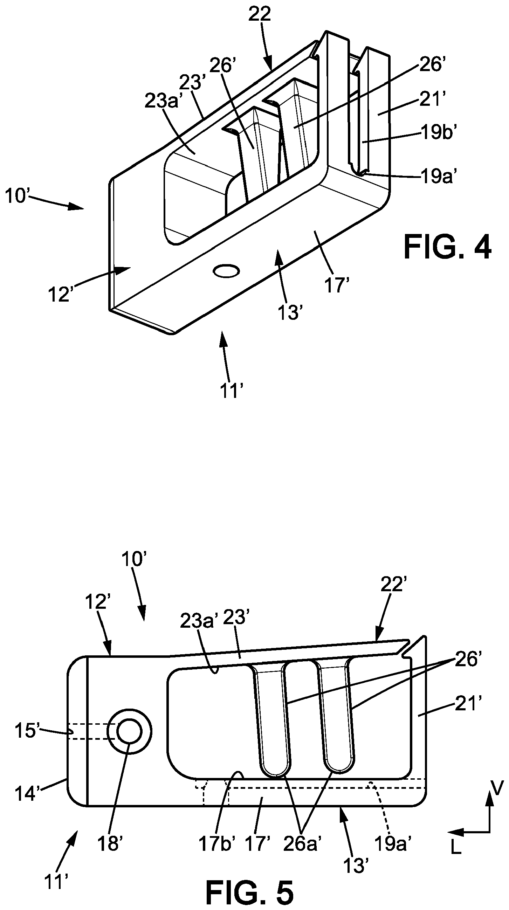

Dans une variante représentée sur les figures 4 et 5, le tendeur 10' comprend également un corps 11' analogue au corps 11 du tendeur 10 précédemment décrit.

Le corps 11' du tendeur 10' selon cette variante comporte un orifice 15' formé dans une surface d'extrémité transversale 14' d'une partie distale 12'. La deuxième extrémité 5b du lien 5 est logée dans l'orifice 15' et maintenue par un organe de serrage monté dans un trou 18' sur une surface latérale de la partie distale 12' du corps 11' de manière déplaçable perpendiculairement à l'axe de l'orifice 15' entre la position de serrage et la position escamotée décrites précédemment.

La partie proximale 13' s'étend selon la direction longitudinale L depuis une surface opposée à la surface d'extrémité 14' de la partie distale 12'. Elle comprend, sur la surface supérieure 17b' de la paroi de base 17', une rainure 19a' adaptée pour recevoir un partie du lien 5 et qui débouche dans une échancrure 19b' ménagée sur toute la hauteur de la paroi transversale 21'.

Le levier de blocage 22', également venu de matière avec le corps 11' réalisé en matière plastique, comporte une ou plusieurs, par exemple deux, pattes de blocage 26' qui s'étendent perpendiculairement depuis la surface inférieure 23a' de la paroi de blocage 23' en regard de la surface supérieure 17b' de la paroi de base 17' de la partie proximale 13' du corps 11'. La patte de blocage 26 est agencée pour que :
- en position ouverte du levier de blocage 22' et à l'état inactif, les extrémités libres 26a' des pattes de blocage 26' soient à distance de la rainure 19a' de la paroi de base 17' de manière à permettre à une portion du lien 5 d'être placée dans la rainure 19a',
- en position fermée du levier de blocage 22 à l'état actif, les extrémités libres 26a' de la patte de blocage 26' s'étendent dans la rainure 19a' de la paroi de base 17' de manière à bloquer la portion du lien 5 placée dans la rainure 19a', les extrémités libres 26a' de la patte de blocage 26' formant des portions de blocage du levier de blocage 22'.

Dans le mode de réalisation représenté, le système d'expansion cutanée 1 comprend en outre une ou plusieurs cales 30 destinées à être interposées entre le lien 5 et les tissus 3 bordant la plaie 2. La cale 30 est réalisée en tout matériau approprié pour permettre une reprise de la traction exercée par le lien 5 sur les tissus 3 bordant la plaie 2 et, le cas échéant, pour permettre d'améliorer le guidage du lien 5 et d'en faciliter la mise en place. En particulier, pour favoriser son adaptation anatomique aux tissus sur lesquels elle repose, la cale 30 peut être réalisée en polymère et notamment en silicone.

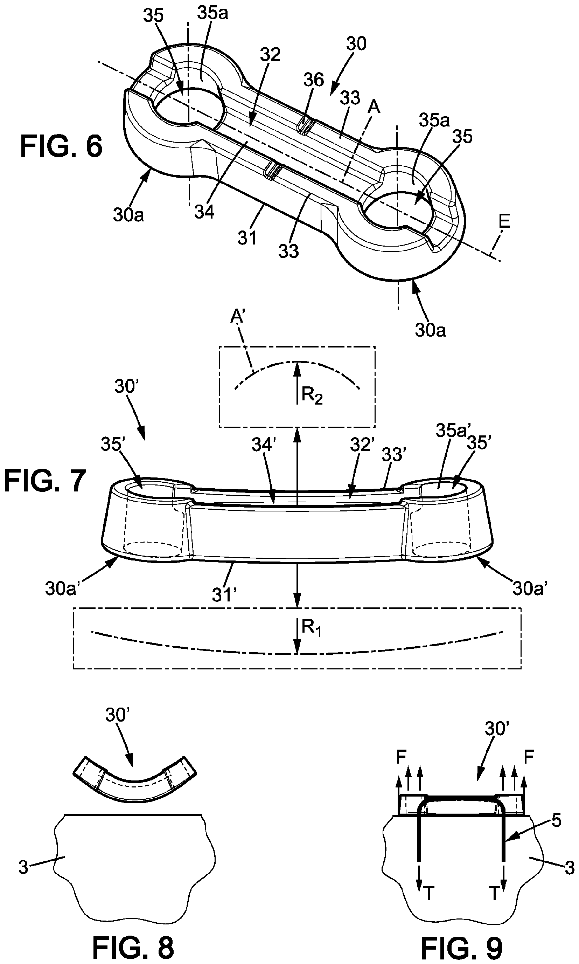

Dans un premier mode de réalisation représenté sur la figure 6, la cale 30 se présente sous la forme d'une plaquette s'étendant selon une direction d'extension E. La cale 30 présente une surface d'appui 31 adaptée pour reposer sur les tissus 3 bordant la plaie 2, et une surface de guidage 32 opposée à la surface d'appui 31 et adaptée pour recevoir une portion du lien 5.

La surface d'appui 31 est plane et dépourvue d'élément d'ancrage adapté pour percer les tissus 3 bordant la plaie 2 et pénétrer dans ces tissus 3. En particulier, la surface d'appui 31 peut être lisse. En variante, tout en étant de préférence dépourvue d'élément d'ancrage, la surface d'appui 31 pourrait être incurvée et/ou être texturée, c'est-à-dire pourvue d'un réseau de saillies non perforantes, pour en améliorer l'adhérence à une surface des tissus 3 bordant la plaie 2.

La surface de guidage 32 s'étend selon un axe de guidage A, dont une partie essentielle est parallèle à la direction d'extension E, et présente une concavité autour de l'axe de guidage A. En particulier, deux rebords longitudinaux 33, parallèles à la direction d'extension E, sont disposés de part et d'autre d'une portion centrale 34 en creux par rapport aux rebords longitudinaux 33.

La cale 30 comporte deux ouvertures de guidage 35 agencées sensiblement à deux extrémités opposées de la cale 30 et adaptées pour permettre un passage du lien 5. Les ouvertures de guidage 35 sont reliées l'une à l'autre par la surface de guidage 32 et présentent chacune une surface latérale 35a, par exemple cylindrique, autour d'un axe central. Une partie de la surface latérale 35a de chacune des ouvertures de guidage 35 forme une extrémité de la surface de guidage 32 de la cale 30. Entre les ouvertures de guidage 35, par exemple dans une zone sensiblement médiane, la cale 30 présente une portion sécable, matérialisée par une rainure 36, permettant de séparer la cale 30 en deux demi-cales 30a comportant chacune l'une des ouvertures de guidage 35 et une partie des surfaces d'appui 31 et de guidage 32.

Dans une variante représentée sur les figures 7 à 9, la cale 30' est conformée de telle manière qu'au repos, en l'absence de contraintes extérieures, la surface d'appui 31' présente une convexité R1 dirigée à l'opposé de la surface de guidage 32'. En outre, la portion centrale 34' de la surface de guidage 32' présente une convexité R2 dirigée à l'opposé de la surface d'appui 31', entre les ouvertures de guidage, et la surface d'appui 31'.

De cette manière, lorsque la cale 30' est positionnée sur les tissus 3 bordant la plaie 2 et que le lien 5 passe entre les ouvertures de guidage 35' sur la surface de guidage 32', la cale 30' peut être déformée pour épouser la surface anatomique des tissus 3 bordant la plaie 2 en exerçant sur le lien 5 une force résultante F opposée à la traction T, dont il résulte une diminution des contraintes sur les tissus 3.

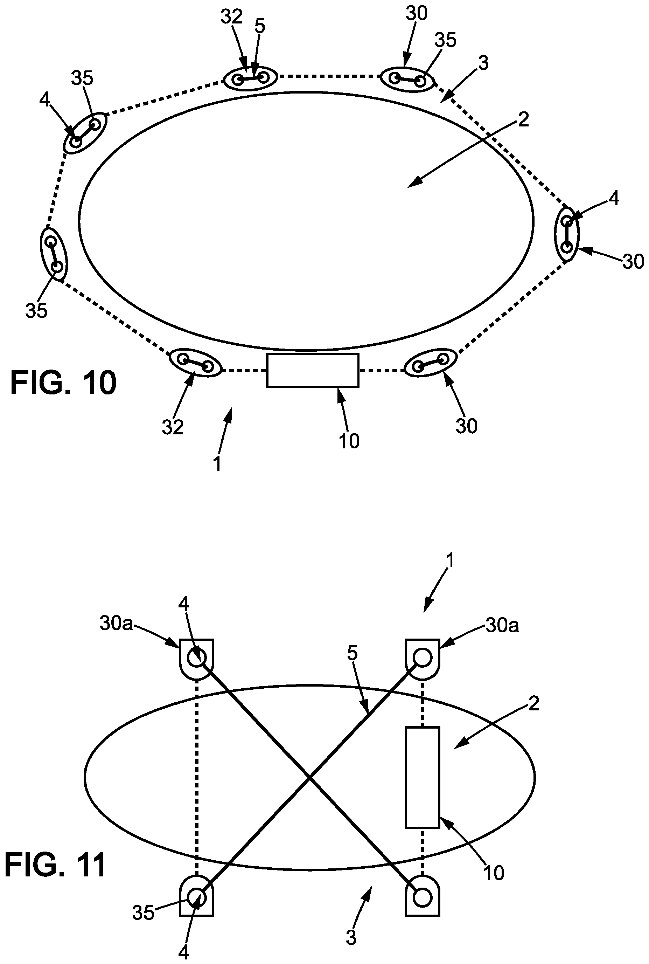

La figure 10 représente une mise en œuvre du système d'expansion cutanée 1 qui vient d'être décrit dans un montage en pont.

La plaie 2 est suturée en passant l'aiguille de suture 6 au travers des tissus 3 bordant la plaie 2, par en-dessous, en un premier point de suture 4 puis au travers de l'une des ouvertures de guidage 35 d'une première cale 30 dont la surface d'appui 31 repose sur des tissus 3 bordant la plaie 2. L'aiguille de suture 6 passe ensuite dans l'autre ouverture de guidage 35 de la première cale 30 puis au travers des tissus 3 bordant la plaie 2, par au-dessus, en un deuxième point de suture 4, le lien 5 passant ainsi au travers des ouvertures de guidage 35 et s'étendant dans la portion centrale 34 de la surface de guidage 32. Cette même opération est répétée pour un nombre déterminé de cales 30 de telle manière que le lien 5 s'étende autour de la plaie 2, en passant sous les tissus 3 bordant la plaie 2 entre deux cales 30 consécutives, et sur la surface de guidage 32 entre les deux ouvertures de guidage 35 de chacune des cales 30.

Une fois que le lien 5 suture convenablement la plaie 2, l'aiguille de suture 6 peut être séparée du lien 5 et le lien 5 peut être introduit dans le passage entre le levier de blocage 22 en position ouverte et le corps 11 du tendeur 10 pour ajuster la tension dans le lien 5. L'orifice 15 retenant la deuxième extrémité 5b du lien 5 et le passage retenant une autre portion du lien 5 étant disposés de part et d'autre du tendeur 10 selon la direction longitudinale L, le tendeur 10 peut être disposé dans l'alignement du lien 5 entre deux points de suture 4 consécutifs.

Lorsqu'une tension satisfaisante est obtenue, le levier de blocage 22 est déplacé vers la position fermée pour bloquer une portion du lien 5 à distance de la deuxième extrémité 5b du lien 5.

Ultérieurement, la tension du lien 5 peut à nouveau être ajustée, et en particulier augmentée, en passant le levier de blocage 22 dans la position ouverte et en tirant sur le lien 5 avant de repasser le levier de blocage 22 dans la position fermée.

Au fur et à mesure que la tension dans le lien 5 augmente, la traction sur les tissus 3 bordant la plaie 2 augmente conduisant à une réduction de la plaie 2.

Le système d'expansion cutanée constitue ainsi un système dynamique pour réduire une plaie 2.

En variante, le système d'expansion cutanée 1 pourrait être mis en œuvre de manière analogue en l'absence de cales 30, le lien 5 suturant directement la plaie en passant alternativement au-dessus et en-dessous des tissus 3 bordant la plaie 2 entre deux points de suture 4 consécutifs. En outre, l'agencement des points de suture 4 et, le cas échéant, des cales 30 pourrait être différent de celui décrit précédemment.

Par exemple, la figure 11 illustre une mise en œuvre du système d'expansion cutanée 1 décrit précédemment dans un montage en lacet. Dans un tel montage, les points de suture 4 ne sont pas joints successivement autour de la plaie 2 comme décrit précédemment mais successivement de part et d'autre de la plaie 2, de sorte que des portions de lien 5 s'étendent sur la plaie 2. En outre, sur la figure 6, les cales 30 ont été séparées en demi-cales 30a, la surface d'appui 31 de l'une des demi-cales 30a reposant sur les tissus 3 bordant la plaie 2 en chaque point de suture 4, le lien 5 passant par l'ouverture de guidage 35 et s'étendant sur la partie de la surface de guidage 32 de la demi-cale 30a.

La tension dans le lien 5 peut être maintenue et ajustée comme décrit précédemment à l'aide du tendeur 10.

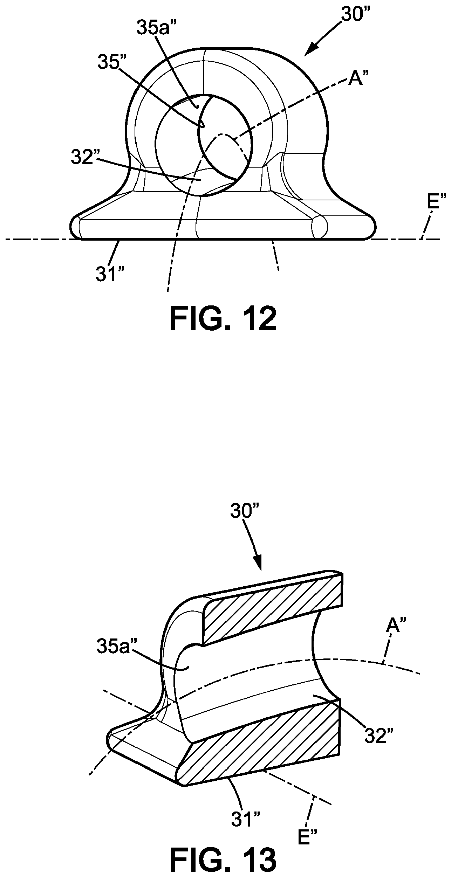

Dans un deuxième mode de réalisation représenté sur les figures 12 et 13, la cale 30" se présente sous la forme d'un plot présentant également une surface d'appui 31" adaptée pour reposer sur les tissus 3 bordant la plaie 2, et une surface de guidage 32" opposée à la surface d'appui 31" et adaptée pour recevoir une portion du lien 5.

La surface d'appui 31" est analogue à celle de la cale 30 selon le premier mode de réalisation.

En revanche, à la différence de la cale 30 selon le premier mode de réalisation, la cale 30" selon le deuxième mode de réalisation comporte une unique ouverture de guidage 35" présentant une surface latérale 35a" autour d'un axe central incurvé autour d'un axe de la surface d'appui 31" parallèle à la direction d'extension E". La surface latérale 35a" est ainsi sensiblement torique et comporte la surface de guidage 32" présentant une forme de paraboloïde hyperbolique avec une concavité autour de l'axe central formant l'axe de guidage A", et une convexité autour de l'axe de la surface d'appui 31".

La plaie 2 est suturée en passant l'aiguille de suture 6 au travers des tissus 3 bordant la plaie 2, par en-dessous, en un premier point de suture 4 puis au travers de l'ouverture de guidage 35" d'une première cale 30" dont la surface d'appui 31" repose sur des tissus 3 bordant la plaie 2. L'aiguille de suture 6 passe ensuite au travers des tissus 3 bordant la plaie 2, par au-dessus, en un deuxième point de suture 4. Cette même opération est répétée pour un nombre déterminé de cales 30" de telle manière que le lien 5 s'étende autour de la plaie 2 ou sur la plaie 2, en passant sous les tissus 3 bordant la plaie 2 entre deux cales 30" consécutives, et sur la surface de guidage 32" de chacune des cales 30". La tension dans le lien 5 peut être maintenue et ajustée comme décrit précédemment à l'aide du tendeur 10.

## Revendications

1. Système d'expansion cutanée (1) pour réduire une plaie (2) bordée par des tissus (3), le système d'expansion cutanée (1) comprenant :
- un lien (5) déformable élastiquement destiné à s'étendre entre les tissus (3) bordant la plaie (2) de telle manière qu'une tension dans le lien (5) produise une traction le long des tissus (3) bordant la plaie (2) pour rapprocher lesdits tissus (3) les uns des autres, le lien (5) présentant des première (5a) et deuxième (5b) extrémités opposées, le lien (5) présentant, au repos une longueur entre les première (5a) et deuxième (5b) extrémités et étant étirable élastiquement de manière à écarter les première (5a) et deuxième (5b) extrémités l'une de l'autre, le lien (5) étant adapté pour suturer la plaie (2) en traversant directement les tissus (3) bordant la plaie (2) en au moins deux points de suture (4), le lien étant réalisé en matériau biocompatible et comprenant une aiguille de suture (6) solidarisée à la première extrémité (5a),
- un tendeur (10) adapté pour maintenir une tension dans le lien (5),
- une pluralité de cales (30 ; 30' ; 30") configurées pour être interposées entre le lien (5) et les tissus (3) bordant la plaie (2) respectivement en une pluralité de points de suture (4), la cale (30 ; 30' ; 30") présentant une surface d'appui (31 ; 31' ; 31") adaptée pour reposer sur les tissus (3) bordant la plaie (2), et une surface de guidage (32 ; 32' ; 32") opposée à la surface d'appui (31 ; 31' ; 31") et adaptée pour recevoir une portion du lien (5),
le système d'expansion cutanée (1) étant **caractérisé en ce que** la surface de guidage (32 ; 32' ; 32") s'étend selon un axe de guidage (A ; A' ; A") et présente une concavité autour de l'axe de guidage (A ; A' ; A").

2. Système d'expansion cutanée (1) selon la revendication 1, dans lequel le lien (5) est réalisé en polymère, et notamment en silicone.

3. Système d'expansion cutanée (1) selon la revendication 1 ou 2, dans lequel le tendeur (10) comprend :
- un corps (11) solidarisé à la deuxième extrémité (5b) du lien (5), et
- un organe de blocage (22) déplaçable par rapport au corps (11) entre un état inactif dans lequel ledit organe de blocage (22) libère un passage pour le lien (5) entre une portion de blocage (22a, 26a) dudit organe de blocage (22) et le corps (11), et un état actif dans lequel ledit organe de blocage (22) maintient une portion du lien (5) à distance de la deuxième extrémité (5b) entre la portion de blocage (22a, 26a) dudit organe de blocage (22) et le corps (11).

4. Système d'expansion cutanée (1) selon l'une quelconque des revendications 1 à 3, dans lequel la surface d'appui (31 ; 31' ; 31") est dépourvue d'élément d'ancrage adapté pour percer les tissus (3) bordant la plaie (2) et pénétrer dans lesdits tissus (3).

5. Système d'expansion cutanée (1) selon l'une quelconque des revendications 1 à 4, dans lequel la cale (30') est conformée de telle manière qu'au repos la surface d'appui (31') présente une convexité (R1) dirigée à l'opposé de la surface de guidage (32').

6. Système d'expansion cutanée (1) selon l'une quelconque des revendications 1 à 5, dans lequel la cale (30 ; 30' ; 30") comporte au moins une ouverture de guidage (35 ; 35' ; 35") présentant une surface latérale (35a ; 35a' ; 35a") autour d'un axe central et adaptée pour permettre un passage du lien (5), la surface latérale (35 ; 35a' ; 35a") formant au moins une partie de la surface de guidage (32 ; 32' ; 32").

7. Système d'expansion cutanée (1) selon la revendication 6, dans lequel la cale (30) comprend au moins deux ouvertures de guidage (35) séparées l'une de l'autre par une portion sécable (36).

8. Système d'expansion cutanée (1) selon l'une quelconque des revendications 1 à 7, dans lequel la cale (30 ; 30' ; 30") est réalisée en polymère, et notamment en silicone.

## Patentansprüche

1. Ein Hautexpansionssystem (1) zum Verkleinern einer Wunde (2), die von Gewebe (3) begrenzt wird, wobei das Hautexpansionssystem (1) beinhaltet:
- ein elastisch verformbares Band (5), das dazu bestimmt ist, sich zwischen den an die Wunde (2) angrenzenden Geweben (3) so zu erstrecken, dass die Spannung des Bandes (5) einen Zug entlang der an die Wunde (2) angrenzenden Gewebe (3) erzeugt, um diese Gewebe (3) einander anzunähern, wobei das Band (5) ein erstes (5a) und ein zweites (5b) entgegengesetztes Ende aufweist, wobei das Band (5) im Ruhezustand eine Länge zwischen dem ersten (5a) und dem zweiten (5b) Ende hat und elastisch dehnbar ist, um das erste (5a) und das zweite (5b) Ende voneinander weg zu bewegen, wobei das Band (5) geeignet ist, die Wunde (2) zu nähen, indem es direkt durch das die Wunde (2) begrenzende Gewebe (3) in mindestens zwei Stichen (4) hindurchgeht, wobei das Band aus biokompatiblem Material besteht und eine am ersten Ende (5a) befestigte Nähnadel (6) aufweist,
- eine Spannvorrichtung (10), die so angepasst ist, dass sie die Spannung im Band (5) aufrechterhält,
- eine Vielzahl von Keilen (30; 30'; 30"), die so konfiguriert sind, dass sie zwischen dem Band (5) und den Geweben (3), die die Wunde (2) begrenzen, jeweils in einer Vielzahl von Stichen (4) angeordnet werden, wobei der Keil (30; 30'; 30") eine Auflagefläche (31; 31'; 31") zum Auflegen auf das Gewebe (3) am Rand der Wunde (2) aufweist, und eine Führungsfläche (32; 32'; 32") gegenüber der Auflagefläche (31; 31'; 31"), die so angepasst ist, dass sie einen Teil des Bandes (5) aufnimmt,
wobei das Hautexpansionssystem (1) **dadurch gekennzeichnet ist, dass** sich die Führungsfläche (32; 32'; 32") entlang einer Führungsachse (A; A'; A") erstreckt und eine Konkavität um die Führungsachse (A; A'; A") herum aufweist.

2. Hautexpansionssystem (1) nach Anspruch 1, bei dem das Band (5) aus Polymer, insbesondere Silikon, besteht.

3. Hautexpansionssystem (1) nach Anspruch 1 oder 2, wobei die Spannvorrichtung (10) beinhaltet:
- einem Körper (11), der am zweiten Ende (5b) des Bandes (5) befestigt ist, und
- ein Verriegelungselement (22), das relativ zu dem Körper (11) zwischen einem inaktiven Zustand, in dem das Verriegelungselement (22) einen Durchgang für das Band (5) zwischen einem Verriegelungsabschnitt (22a, 26a) des Verriegelungselements (22) und dem Körper (11) freigibt, und einem aktiven Zustand, in dem das Verriegelungselement (22) einen Abschnitt des Bandes (5) von dem zweiten Ende (5b) zwischen dem Verriegelungsabschnitt (22a, 26a) des Verriegelungselements (22) und dem Körper (11) entfernt hält, beweglich ist.

4. Hautexpansionssystem (1) nach einem der Ansprüche 1 bis 3, wobei die Auflagefläche (31; 31'; 31") frei von Verankerungselementen ist, die geeignet sind, das an die Wunde (2) angrenzende Gewebe (3) zu durchstechen und zu durchdringen.

5. Hautexpansionssystem (1) nach einem der Ansprüche 1 bis 4, wobei der Keil (30') so geformt ist, dass die Auflagefläche (31') im Ruhezustand eine von der Führungsfläche (32') weg gerichtete Konvexität (R1) aufweist.

6. Hautexpansionssystem (1) nach einem der Ansprüche 1 bis 5, wobei der Keil (30; 30'; 30") mindestens eine Führungsfläche (35; 35'; 35") beinhaltend eine Seitenfläche (35a; 35a'; 35a") um eine Mittelachse aufweist, geeignet den Durchgang des Bandes (5) zu ermöglichen, wobei die Seitenfläche (35; 35a'; 35a") mindestens einen Teil der Führungsfläche (32; 32'; 32") bildet.

7. Hautexpansionssystem (1) nach Anspruch 6, wobei der Keil (30) mindestens zwei Führungsöffnungen (35) aufweist, die durch einen zerbrechlichen Abschnitt (36) voneinander getrennt sind.

8. Hautexpansionssystem (1) nach einem der Ansprüche 1 bis 7, wobei der Keil (30; 30'; 30") aus Polymer, insbesondere Silikon, hergestellt ist.

## Claims

1. Skin expansion system (1) to reduce a wound (2) surrounded by tissues (3), the skin expansion system (1) comprising:
- an elastically deformable tie (5) intended to extend between the tissues (3) surrounding the wound (2) in such a way that tension in the tie (5) produces traction along the tissues (3) surrounding the wound (2) in order to bring said tissues (3) closer together, the tie (5) having opposed first (5a) and second (5b) ends, the tie (5) having at rest a length between the first (5a) and second (5b) ends and being elastically stretchable to move the first (5a) and second (5b) ends away from each other, the tie (5) is adapted to suture the wound (2) by passing directly through the tissues (3) surrounding the wound (2) at at least two suture points (4), the tie being made from a biocompatible material and comprising a suture needle (6) rigidly connected to the first end (5a),
- a tensioner (10) adapted to maintain tension in the tie (5),
- a plurality of spacers (30; 30'; 30") configured to be interposed between the tie (5) and the tissues (3) surrounding the wound (2) at a plurality of suture points, the spacer (30; 30'; 30") having a bearing surface (31; 31'; 31") adapted to rest on the tissues (3) surrounding the wound (2), and a guide surface (32; 32'; 32") opposite the bearing surface (31; 31'; 31") and adapted to receive a portion of the tie (5),
the skin expansion system (1) being **characterised in that** the guide surface (32; 32'; 32") extends along a guide axis (A; A'; A") and is concave around the guide axis (A; A'; A").

2. Skin expansion system (1) according to claim 1, wherein the tie (5) is made of a polymer, and particularly silicone.

3. Skin expansion system (1) according to claim 1 or 2, wherein the tensioner (10) comprises:
- a body (11) fixed to the second end (5b) of the tie (5), and
- a blocking device (22) that can be moved relative to the body (11) between an inactive state in which said blocking device (22) releases a passage for the tie (5) between a blocking portion (22a, 26a) of said blocking device (22) and the body (11), and an active state in which said blocking device (22) keeps a portion of the tie (5) at a distance from the second end (5b) between the blocking portion (22a, 26a) of said blocking device (22) and the body (11).

4. Skin expansion system (1) according to any one of claims 1 to 3, wherein the bearing surface (31; 31'; 31") does not have an anchor member adapted to pierce the tissues (3) surrounding the wound (2) and penetrate into these tissues (3).

5. Skin expansion system (1) according to any one of claims 1 to 4, wherein the spacer (30') is shaped such that when at rest, the bearing surface (31') has a convexity (R1) in the direction opposite to the guide surface (32').

6. Skin expansion system (1) according to any one of claims 1 to 5, wherein the spacer (30; 30'; 30") comprises at least one guide opening (35; 35'; 35") with a lateral surface (35a; 35a'; 35a") around a central axis and adapted to enable the tie (5) to pass through, the lateral surface (35a; 35a'; 35a") forming at least part of the guide surface (32; 32'; 32").

7. Skin expansion system (1) according to claim 6, wherein the spacer (30) comprises at least two guide openings (35) separated from each other by a break-off portion (36).

8. Skin expansion system (1) according to any one of claims 1 to 7, in which the spacer (30; 30'; 30") is made of a polymer, and particularly silicone.
